(19) Europäisches Patentamt
European Patent Office
Office européen des brevets

(11) **EP 3 087 943 A1**

(12) **EUROPEAN PATENT APPLICATION**

(43) Date of publication:
**02.11.2016 Bulletin 2016/44**

(51) Int Cl.:
*A61B 18/14* (2006.01)  *A61B 18/00* (2006.01)

(21) Application number: **16167580.6**

(22) Date of filing: **28.04.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.04.2015 US 201562154436 P**

(71) Applicant: **Cook Medical Technologies LLC
Bloomington, IN 47404 (US)**

(72) Inventors:
• **SIGMON, John Crowder Jr.**
**Winston-Salem, NC 27104 (US)**
• **SATHYANARAYAN, Deepak**
**Ormond Beach, FL 32174 (US)**
• **MURRAY, Kristin**
**Omaha, Nebraska 68102 (US)**

(74) Representative: **Mathys & Squire LLP
The Shard
32 London Bridge Street
London SE1 9SG (GB)**

(54) **ELECTROSURGICAL DEVICE**

(57) An electrosurgical medical device may include an elongate needle that is delivered to a gastrointestinal tract of a patient and inserted through the gastrointestinal wall and into a cyst. Fluid within the cyst may be aspirated through a lumen of the needle. The cyst may then be filled with conductive fluid by delivering the conductive fluid through the needle lumen. Radio frequency energy may then be delivered to the needle and transferred to the cyst to ablate the cyst. A handle assembly of the electrosurgical medical device may communicate the cyst and conductive fluids to and from the needle lumen, as well as communicate radio frequency energy to the needle and a temperature signal generated by a thermocouple. An electrical cable assembly adapted to communicate both the radio frequency energy and the temperature signal may be removably connectable with the handle assembly.

FIG. 1A

EP 3 087 943 A1

# Description

## TECHNICAL FIELD

The present invention relates generally to medical devices and more particular to endoscopic medical devices, systems and methods for ablating cysts, and also to a handle assembly and an electrical cable assembly that integrate fluid delivery, RF energy delivery, and temperature sensing components.

## BACKGROUND

The current standard of care for treating cysts is a "wait and watch" approach in which a patient undergoes periodic X-ray computer tomography scans (or CT scans) to monitor the size of the cyst. If the cyst grows to or above a certain size threshold, usually around three centimeters (which is typically indicative of malignancy) for a pancreatic cyst, invasive or open surgery may be performed to physically remove the cyst from the body. Such open surgery may involve making an incision into the patient's skin in order to gain access into his/her abdominal cavity. A much less or minimally invasive procedure may be desirable, not only to treat patients having a cyst that has grown to the size threshold, but also to treat patients with cysts that have not yet grown to the size threshold.

## BRIEF SUMMARY

The present description describes a handle assembly that integrates fluid delivery, radio frequency (RF) energy, and temperature sensing components and an associated electrical cable assembly. The handle assembly and the electrical cable assembly may be configured to be removably connectable with each other. The present description also describes endoscopic medical devices, systems, and methods for ablating cysts. In one embodiment, a method of ablating a cyst is performed. The method includes delivering a distal portion of a needle of an electrosurgical medical device to a gastrointestinal tract location in a patient; from the gastrointestinal tract location, advancing the distal portion of the needle to within the cyst; and while the distal portion of the needle is within the cyst, transferring a predetermined amount of radio frequency (RF) energy from the distal portion of the needle to the cyst to ablate the cyst.

In some embodiments, the predetermined amount of RF energy is within a range from 165 Joules to 11,250 Joules.

In some embodiments, the predetermined amount of RF energy is between 165 Joules and 275 Joules when the cyst has a diameter of about one centimeter, the predetermined amount of RF energy is between 900 Joules and 1,500 Joules when the cyst has a diameter of about two centimeters, and the predetermined amount of RF energy is between 6,750 and 11,250 Joules when the cyst has a diameter of three centimeters.

In some embodiments, transferring the predetermined amount of RF energy comprises transferring the predetermined amount of RF energy from the distal portion of the needle to the cyst via a conductive fluid inside the cyst.

In some embodiments, the needle comprises a hollow needle comprising a needle lumen extending through a needle body of the needle, and the method further includes: delivering the conductive fluid to inside the cyst via the needle lumen of the needle.

In some embodiments, the method further includes aspirating cyst fluid inside the cyst through the needle lumen before delivering the conductive fluid to inside the cyst.

In some embodiments, the electrosurgical device comprises a handle assembly coupled to a hollow needle, and the method further includes: aspirating cyst fluid through a coupling member lumen of a coupling member that couples the hollow needle to a housing of the handle assembly; delivering the conductive fluid through the coupling member lumen; and delivering the predetermined amount of RF energy through the coupling member.

In some embodiments, the method further includes: sensing, with a thermocouple, a temperature inside the cyst; and transmitting, with the thermocouple, a temperature signal indicative of the sensed temperature to the handle assembly.

In some embodiments, the method further includes: transmitting the predetermined amount of RF energy from the needle body to a base of the handle assembly, the base supporting a first contact engaged with an electrical cable assembly delivering the predetermined amount of RF energy from a power source to the first contact; and transmitting, with the thermocouple, the temperature signal to the base, the base further supporting second and third contacts also engaged with the electrical cable assembly, the electrical cable assembly further delivering the temperature signal to a temperature measurement device.

In another embodiment, an electrosurgical medical device includes a handle assembly and an elongate tubular member. The handle assembly includes: a handle assembly housing; a fluid delivery channel extending within the handle assembly housing; and a radio frequency (RF) energy delivery path extending within the handle assembly housing. The elongate tubular member extends from a proximal portion to a distal portion includes a conductive body and a lumen extending in the conductive body. The conductive body is electrically coupled to the RF energy delivery path and the lumen is in fluid communication with the fluid delivery channel.

In some embodiments, a coupling member couples the conductive body to the handle assembly housing. The coupling member includes a coupling member lumen that forms part of the fluid delivery channel.

In some embodiments, the coupling member comprises a conductive material and forms part of RF energy delivery path such that the elongate tubular member is both electrically coupled to the RF energy delivery path and in fluid communication with the fluid delivery channel via the coupling member.

In some embodiments, the handle assembly includes a conductive donut-shaped member that is part of the RF energy delivery path and disposed about a proximal portion and in contact with a shoulder of the coupling member.

In some embodiments, a thermocouple includes a distal end disposed at a distal portion of the elongate tubular member and proximally extends to within the handle assembly housing.

In some embodiments, the thermocouple extends through at least a portion of the coupling member lumen.

In some embodiments, the handle assembly includes a handle-side connection area adapted for removable connection with an electrical cable assembly. The handle-side connection area includes a portion of the handle assembly housing and a plurality of handle-side electrical contacts integrated with the portion of the handle assembly housing. The plurality of handle-side electrical contacts includes a first handle-side contact that is part of the RF energy delivery path and second and third handle-side contacts that are in electrical communication with the thermocouple.

In some embodiments, a second channel extends in the handle assembly housing, and the thermocouple and a conductive wire that is part of the RF energy delivery path extend in the second channel.

In some embodiments, the handle assembly includes a base that supports the plurality of handle-side electrical contacts, and the second channel extends in the housing to the base.

In some embodiments, a conductive donut-shaped member is part of the RF energy delivery path and includes a planar surface biased against an internal surface of the handle assembly housing. The conductive wire extends through a gap in the internal surface in order to be connected to the conductive donut-shaped member.

In some embodiments, an electrical cable assembly includes a connector comprising a connector housing and a cable-side connection area adapted for removable connection with the handle-side connection area. The cable-side connection area includes a portion of the connector housing integrated with a plurality of cable-side electrical contacts. In addition, the plurality of cable-side electrical contacts comprises a first cable-side contact designated for removable connection with the first handle-side contact, and second and third cable-side contacts designated for removable connection with the second and third handle-side contacts.

In some embodiments, the handle-side connection area and the cable-side connection area form a plug and socket configuration.

In some embodiments, the handle-side connection area and the cable-side connection area include matching cross-sectional shapes that are symmetrical with respect to a single axis.

In some embodiments, the electrical cable assembly includes a first elongate conductive member electrically connected to the first cable-side contact and terminates with a first plug adapted for removable connection with a power source configured to generate the RF energy. In addition, the electrical cable assembly includes a second elongate conductive member electrically connected to the second and third cable-side contacts. The second elongate conductive member terminates with a second plug adapted for removable connection with a temperature measurement device.

In some embodiments, the elongate tubular member comprises a needle.

In another embodiment, an electrosurgical medical device includes: an elongate conductive member extending from a proximal portion to a distal portion; a thermocouple; a handle assembly coupled to the elongate conductive member, and an electrical cable assembly. The handle assembly includes a handle assembly housing, a radio frequency (RF) energy delivery path extending within the handle assembly housing and electrically coupled to the elongate conductive member, and a plurality of handle-side electrical contacts. The electrical cable assembly includes: a connector housing, and a cable-side connection area that comprises a plurality of cable-side electrical contacts integrated with the connector housing. The plurality of cable-side electrical contacts are configured for removable connection with the plurality of handle-side contacts, and when the plurality of cable-side contacts are connected to the plurality of handle-side contacts, the electrical cable assembly is electrically coupled to the RF energy delivery path and the thermocouple.

In some embodiments, the handle assembly includes a handle-side connection area adapted for removable connection with the cable-side connection area. The handle-side connection area includes a portion of the handle assembly housing and the plurality of handle-side electrical contacts integrated with the portion of the handle assembly housing.

In some embodiments, the handle-side connection area and the cable-side connection area form a plug and socket configuration.

In some embodiments, the handle-side connection area and the cable-side connection area include matching cross-sectional shapes that are symmetrical with respect to a single axis.

In some embodiments, the electrical cable assembly includes a first elongate conductive member configured to be electrically coupled to the RF energy delivery path and terminating with a first connector adapted for removable connection with a power source configured to generate the RF energy, and a second elongate conductive configured to be electrically coupled to the thermocouple and terminating with a second connector adapted for removable connection with a temperature measurement device.

In some embodiments, the handle assembly further includes a fluid delivery channel extending within the handle assembly housing, and the elongate conductive member is electrically coupled to the RF energy delivery path and in fluid communication with the fluid delivery channel.

In some embodiments, the handle assembly includes a coupling member that couples the elongate conductive member to the handle assembly housing, wherein the coupling member includes a coupling member lumen that forms part of the fluid delivery channel.

In some embodiments, the coupling member comprises a conductive material and forms part of RF energy delivery path. The elongate conductive member is both electrically coupled to the RF energy delivery path and in fluid communication with the fluid delivery channel via the coupling member.

In some embodiments, the thermocouple extends through at least a portion of the coupling member lumen.

In some embodiments, the elongate tubular member comprises a needle.

Other embodiments are possible, and each of the embodiments can be used alone or together in combination. Accordingly, various embodiments are now described with reference to the attached drawings.

## BRIEF DESCRIPTION OF THE DRAWINGS

Fig. 1A is a partial cross-sectional side view of an example endoscopic medical system with a distal portion located near a cyst to be ablated.

Fig. 1 B is a partial cross-sectional side view of the endoscopic medical system of Fig. 1A, where a distal tip of a needle is inserted into the cyst.

Fig. 1C is a partial cross-sectional side view of the endoscopic medical system of Fig. 1A, where fluid in the cyst is aspirated through the needle.

Fig. 1 D is a partial cross-sectional side view of the endoscopic medical system of Fig. 1A, where a fluid source containing conductive fluid is attached to a handle assembly.

Fig. 1 E is a partial cross-sectional side view of the endoscopic medical system of Fig. 1A, where the conductive fluid is inserted into the cyst.

Fig. 1 F is a partial cross-sectional side view of the endoscopic medical system of Fig. 1 A, where radio frequency energy is transferred to the cyst to ablate the cyst.

Fig. 2 is a cross-sectional side view of example components of a handle assembly of the medical system of Fig. 1A.

Fig. 3 is a cross-sectional side view of an example configuration of electrical cabling assembly and example housing of the handle assembly of Fig. 1A.

Fig. 4 is a cross-sectional side view of the components shown in Fig. 2 in combination with the housing shown in Fig. 3.

Fig. 5 is a cross-sectional axial view of the housing of taken long lines 5-5 of Fig. 3.

Fig. 6 is a cross-sectional axial view of the housing and conductive pins taken along lines 6-6 of Fig. 4.

Fig. 7 is a cross-sectional axial view of a connector and its conductive receptacles taken along lines 7-7 of Fig. 4.

Fig. 8 is a cross-sectional side of another example configuration of components of the handle assembly of Fig. 1A.

Fig. 9 is a plot showing an amount of radio frequency energy to be applied to a cyst as a function of the diameter of the cyst.

## DETAILED DESCRIPTION

The present description describes a handle assembly that integrates fluid delivery, radio frequency (RF) energy, and temperature sensing components and an associated electrical cable assembly. The handle assembly and the electrical cable assembly may be configured to be removably connectable with each other. The present description also describes endoscopic medical devices, systems, and methods for ablating cysts. The handle assembly and the electrical cable assembly may be components of the medical devices and systems and used to ablate the cysts. However, the handle assembly and electrical cable assembly may also or alternatively be used with medical devices and systems that perform electrosurgical procedures that involve fluid delivery, RF energy delivery, and temperature sensing other than cyst ablation.

A cyst is a closed sac having a membrane that encloses and contains air, fluid, or other semisolid material therein. Cysts may form on various parts of the body, such as the pancreas, liver, bile duct, lungs, diaphragm, and spleen, as examples. Cysts may be detected through X-ray computer tomography (X-ray CT or simply CT) scans. Typically, a patient undergoes periodic CT scans. Tomographic images produced from the CT scans may identify a cyst, where on or within the body the cyst is located, and the size (e.g., diameter) of the cyst. When a cyst is detected, its size may be monitored in order to determine whether the cyst should be removed. As long as the cyst remains smaller than a predetermined size, such as three centimeters in diameter for a pancreatic cyst, the physician monitoring the cyst may determine not to remove the cyst. However, if the cyst grows to the predetermined size and/or continues to grow beyond the predetermined size, the physician may determine to remove the cyst.

As mentioned in the background section, when a physician determines to remove the cyst, the physician may perform an invasive procedure. In particular, open surgery may be performed in which an incision in the patient's skin may be made in order to gain access into the patient's abdominal cavity. Once access is obtained, the cyst may be physically removed or detached from the body.

Rather than perform open surgery, the present description describes an endoscopic ablation procedure that may be performed to gain access to and remove the cyst. The endoscopic ablation procedure may be a minimally invasive procedure in which an incision into the patient's stomach to gain access into the abdominal cavity is not performed. Instead, an elongate tubular portion of an endoscope may be inserted into the patient's body endoscopically, such as into the mouth and moved through the esophagus until it reaches an area or location of the patient's gastrointestinal (GI) tract near the cyst and/or that has been identified as being advantageous for accessing the cyst. A distal tip of a hollow needle may then be endoscopically delivered (i.e., delivered through a working channel or lumen of the endoscope) to the GI tract area where the distal end of the endoscope is positioned. The distal tip may then be inserted through the GI wall and the membrane of the cyst to inside the cyst. After the distal tip is inserted into the cyst, fluid inside the cyst may be aspirated through the distal tip. After the fluid is aspirated, conductive fluid, such as saline, may be delivered through the distal tip into the cyst. RF energy may then be delivered to the distal tip, which may be transferred to the cyst membrane via the conductive fluid surrounding the distal tip. Application of the RF energy to the cyst may ablate the cyst membrane. In particular, the tissue may heat up in response to receiving the RF energy, killing the cyst membrane after passage of an amount of time. In sum, rather than physically excise the cyst membrane from the body, the cyst membrane may be removed by ablating it through application of RF energy.

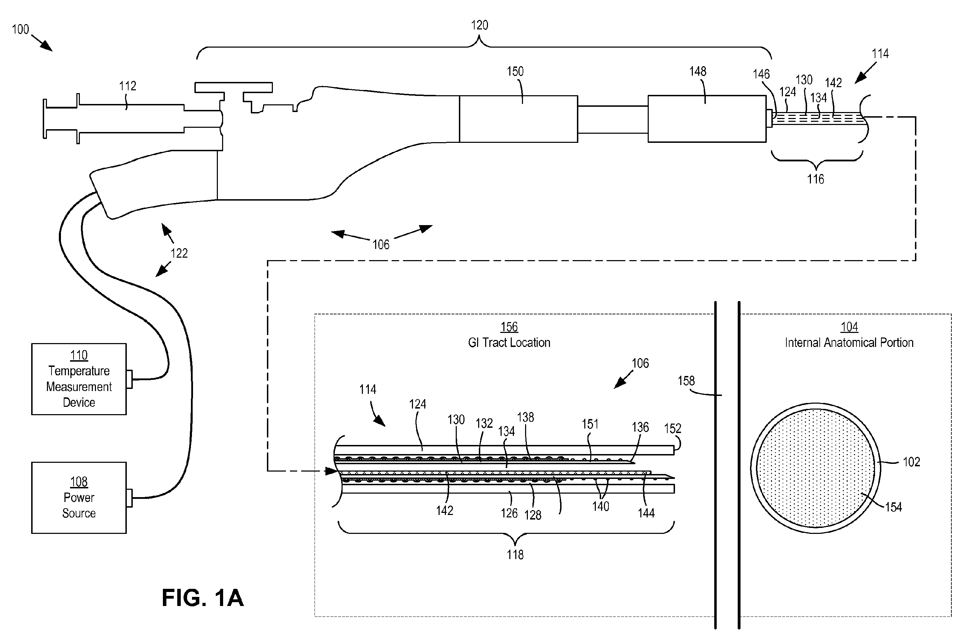

Fig. 1 A shows a partial cross-sectional side view of an example endoscopic medical system 100 that may be used to endoscopically access and ablate a cyst 102 located on or within an internal anatomical portion 104, such a tissue or an internal organ, of a patient. As described in further detail below, the internal anatomical portion 104 may be adjacent or near a location 156 of the patient's GI tract that is accessible with the endoscopic medical system 100. The endoscopic medical system 100 may include an electrosurgical device 106 configured to be coupled to a power source 108, temperature measurement device 110, and one or more syringes 112 (or other pumps). The power source 108 may be an electronic device, such as a radio frequency (RF) generator or an electrosurgical unit (ESU) that is configured to generate and supply electrical current, including RF electrical current, to the electrosurgical device 106. The temperature measurement device 110 may be configured to measure or calculate a temperature based on an electrical signal indicative of temperature received from the electrosurgical device 106. The syringe 112 may be configured to take in liquid received from and expel liquid delivered to the electrosurgical device 106.

The electrosurgical device 106 may include an elongate insertion portion 114 for insertion into the patient that extends from a proximal portion 116 to a distal portion 118. The electrosurgical device 106 may also include a handle assembly 120 operatively coupled to the elongate insertion portion 114 and that a physician or operator may handle to control operation of the electrosurgical device 106. The electrosurgical device 106 may further include or be configured to be coupled to an electrical cable assembly 122 that electrically couples the power source 108 and the temperature measurement device 110 to the handle assembly 120.

The elongate insertion portion 114 may include an elongate tubular member 124, such as a catheter, that includes a body 126 and a lumen 128 longitudinally extending through the body 126. The elongate insertion portion 114 may further include a hollow needle 130 that longitudinally extends and is movably disposed within the lumen 128 of the elongate tubular member 124. The needle 130 may include a body 132 made of a conductive material (e.g., stainless steel) and a needle lumen 134 extending through the needle body 132. For some example configurations, the needle 130 may be a twenty-two gauge needle, although other sizes for the needle 130 may be possible. The hollow needle 130 may include a sharp and/or beveled distal tip 136. In addition, an outer surface 138 of the needle body 132 may include dimples 140, which may enhance ultrasound visibility and/or guidance of the needle 130 during operation.

The elongate insertion portion 114 may further include a thermocouple 142 configured to sense a temperature of the environment at and/or surrounding a distal end 144 of the thermocouple 142. As shown in Fig. 1A, the distal end 144 of the thermocouple 142 may be located proximate to the distal tip 136 of the needle 130 at the distal portion 118 of the elongate insertion portion 114. The thermocouple 142 may include a pair of conductors made of different or dissimilar metals. At the distal end 144, the conductors may be in contact with each other, such as by being welded together for example. Proximal the distal end 144, the conductors may each be encapsulated with an insulated material, such as a polymer, so that they are electrically insulated from each other. In addition, for some example configurations, the encapsulated conductors may extend from the proximal portion 116 to the distal portion 118 as a twisted pair.

Also, as shown in Fig. 1A, for some example configurations, the thermocouple 142 may longitudinally extend from the proximal portion 116 to the distal portion 118 within the needle lumen 134, although other ways in which the thermocouple 142 may extend from the proximal portion 116 to the distal portion 118 may be possible. At the distal end 144 where the conductors are in contact with each other, the conductors may generate a signal, such as a voltage signal, indicative of a temperature differential when a temperature of the environment surrounding the distal end 144 differs from a reference temperature. A level, such as a voltage level, of the temperature signal may indicate the sensed temperature. As described in further detail below, the temperature signal may be sent back to the temperature measurement device 110, which may measure or calculate the temperature indicated by the level of the temperature signal.

The handle assembly 120 may be coupled to a proximal end 146 of the elongate insertion portion 114. The handle assembly 120 may include a portion operatively coupled to the elongate tubular member 124 and the needle 130 and configured to longitudinally move the elongate tubular member 124 relative to the needle 130. In the example configuration shown in Fig. 1A, the portion of the handle assembly 120 may include a first member 148 configured to move relative to a second member 150 in order to longitudinally move the elongate tubular member 124 relative to the needle 130. The first and second members 146, 148 may be moved relative to each other in order to advance a distal portion 151, including the distal tip 136, of the needle 130 past a distal end 152 of the elongate tubular member 124 in order to expose the distal tip 136 to its outer surroundings and to withdraw the distal tip 136 back to within the tubular member 124.

As described in further detail below, fluid 154 contained within the cyst 102 may be drained from the cyst 102 by being aspirated through the needle lumen 134 and sent to the syringe 112. After the cyst fluid 154 is aspirated, a conductive fluid, such as saline, may be delivered through the needle lumen 134 into the cyst 102. Subsequently, RF energy may be delivered to the distal tip 136 of the needle 130 and transferred to the cyst 102 via the conductive fluid in order to ablate the cyst 102.

The handle assembly 120 may be configured to deliver fluid, including the cyst fluid and the conductive fluid between the syringe 112 and the needle lumen 134. In addition, the handle assembly 120 may be configured to deliver RF energy from the electrical cable assembly 122 to the needle 130 and to deliver temperature signals generated by the thermocouple 142 to the electrical cable assembly 122. To do so, the handle assembly 120 may be configured to engage with and/or couple to a proximal end or hub of the needle body 132 such that the needle lumen 134 is in fluid communication with a fluid delivery channel of the handle assembly 120, and at the same time the needle body 134 is electrically coupled to a portion of the handle assembly 120 that is configured to be electrically coupled to the power source 108. The handle assembly 120 may also be configured such that while the proximal end or hub of the needle body 132 is engaged with and/or coupled to the handle assembly 120, the thermocouple 142 may extend through the needle lumen 134 and proximally to within the handle assembly 120, where a proximal end of the thermocouple 142 is electrically connected to a portion of the handle assembly 120 that is configured to be electrically coupled to the temperature measurement device 110.

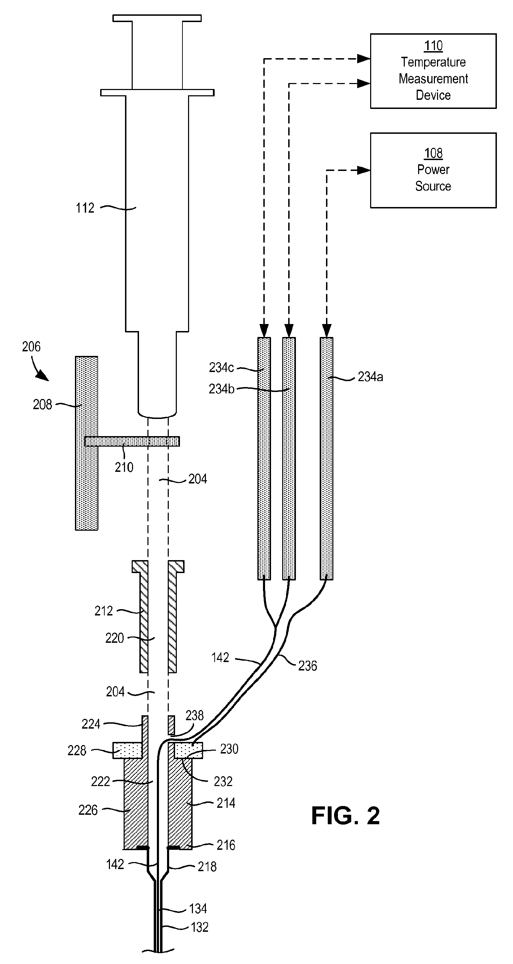

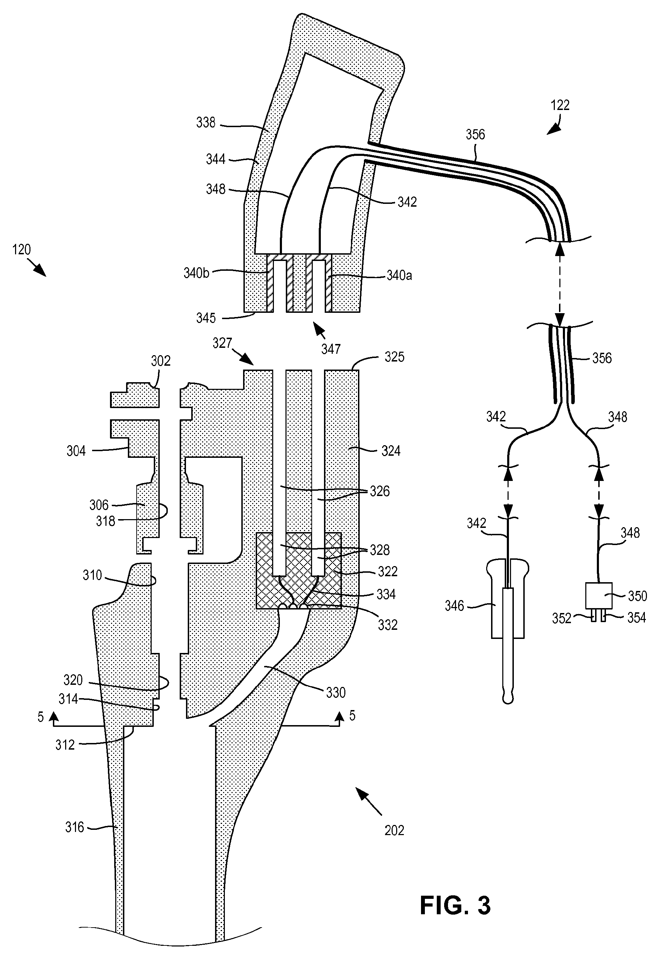

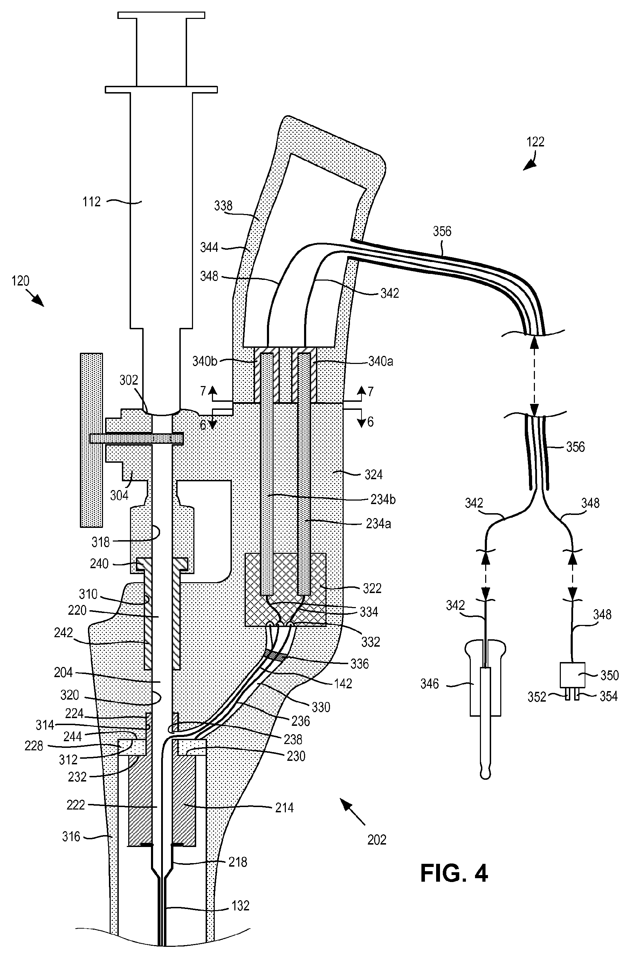

Figs. 2-4 show example configurations of the handle assembly 120 and the electrical cable assembly 122 in further detail. Fig. 2 shows a cross-sectional side view of example components of the handle assembly 120 that deliver fluid between the syringe 112 and the needle lumen 132, deliver RF energy between the electrical cable assembly 122 and the conductive needle body 132, and deliver temperature signals between the electrical cable assembly 122 and the thermocouple. Components that deliver the RF energy may be considered components of an RF energy delivery path of the handle assembly 120. Fig. 3 shows a cross-sectional side view of an example configuration of housing 202 of the handle assembly 120 that houses and/or supports the components shown in Fig. 2. Fig. 3 also shows an example configuration of the electrical cable assembly 122. Fig. 4 shows the components shown in Fig. 2 in combination with the housing 202 and example configuration of the electrical cable assembly 122 shown in Fig. 3.

Referring particularly to Fig. 2, a fluid delivery channel 204 configured to deliver fluid between the syringe 212 and the needle lumen 134 may extend in the handle assembly 120. The handle assembly 120 may include a valve 206, such as a stopcock or other ball valve, that is configured to control the flow of fluid through the fluid delivery channel 204. The valve 206 may include a handle or lever 208 and a stem 210 connected to the valve handle 208 and extending to the fluid delivery channel 204. The valve handle 208 may be operated to rotate the valve stem 210 so that the valve stem 210 either allows or prevents the flow of fluid from the syringe 112 to a portion of the fluid delivery channel 204 distal the valve stem 210, or vice versa.

The handle assembly 120 may also include a luer 212 and a coupling member 214 that couples and/or fixedly attaches the needle body 132 to the handle assembly 120. In some example configurations, each of the luer 212 and the coupling member 214 may be generally cylindrical structures, although other shaped structures may be possible. A distal end 216 of coupling member 214 may be connected to a proximal needle hub 218 of the needle body 132. By being connected to the needle hub 218, the coupling member 214, and the handle assembly 120 in general, may be in fluid communication with the needle 130. Each of the luer 212 and the coupling member 214 may include a respective lumen 220, 222. The lumens 220, 222 may each form a part of the fluid delivery channel 204.

The luer 212 may be made of a non-conductive material, such as plastic, and serve as a bridge between the valve 206 and the coupling member 214. Fluid passing from the syringe 212 through the valve stem 210 may also pass through the luer lumen 220 before flowing through the coupling member lumen 222. Similarly, fluid passing through the coupling member lumen 222 toward the syringe 112 may first pass through the luer lumen 220 before reaching the valve stem 210 and the syringe 112.

As mentioned, the coupling member 214 may be configured to couple and/or fixedly attach the needle body 132 to the handle assembly. To do so, the coupling member 214 may include a proximal portion 224 configured to couple to the housing 202 of the handle assembly 120 and a distal portion 226 configured to couple to the needle hub 218. In addition, the example configurations of the handle assembly 120 shown in Figs. 2-4 utilize a conductive washer or other donut-shaped disc-like structure 228 for part of the RF delivery path and also to enhance the coupling between the coupling member 214 and the housing 202. In particular, the washer 228 may be circumferentially disposed about the proximal portion 224, and a transition between the proximal portion 224 and the distal portion 226 may provide a shoulder 230 on which a first planar surface 232 of the washer 228 may be disposed and come into contact. For this configuration, the distal portion 226 may have an outer diameter that is greater than an outer diameter of the proximal portion 224, and an inner diameter of the metal washer 228 may be larger than the outer diameter of the proximal portion 224 of the coupling member 214 and smaller than the outer diameter of the distal portion 226.

In addition, the handle assembly 120 may include a set of three conductive pins 234a, 234b, 234c configured to contact corresponding electrical contacts of the electrical cable assembly 122 in order to form an electrical connection between the handle assembly 120 and the electrical cable assembly 122. A wire 236 or other elongate conductive member may be connected to the washer 228 in order to electrically couple the washer 228 with a first pin 234a configured to deliver RF energy generated by the power source 108. The other two conductive pins, 234b and 234c, may be configured to communicate the temperature signals ultimately communicated to the temperature measurement device 110.

As shown in Fig. 2, the coupling member 214 may include a gap or slit 238. The gap 238 is shown extending in the proximal portion 224, although in other configurations, the gap 238 may extend in the distal portion 226. A proximal portion of the thermocouple 142 may proximally extend through a portion of the needle lumen 134 circumferential with the proximal needle hub 218, through the coupling member lumen 222, and then through the gap 238. Once outside the needle lumen 134 and the coupling member lumen 222, the proximal portion of the thermocouple 142 may proximally extend away from the coupling member 214 and toward the conductive pins 234b, 234c. As shown in Fig. 2, at the proximal end of the thermocouple 142, each of the conductors of the thermocouple 142 may be electrically connected to a respective one of the conductive pins 234b, 234c.

Referring to Figs. 3 and 4, the housing 202 may include a first portion 302 configured to engage and provide a removable connection with the syringe 112, and a second portion 304 configured to house and support the valve 206. The housing 202 may further include a third portion 306 configured to house and support a proximal end or lip 240 of the luer 212. In addition, the housing 202 may include a fourth portion 310 configured to engage with and support a distal portion 242 of the luer 212. For some example configurations, the fourth portion 310 and the distal portion 242 may be configured to be threadingly engaged with each other. For example, the distal portion 242 may be screwed into the fourth portion 310. The housing 202 may further include an inner surface 312 facing in the distal direction on which a second planar surface 244 of the washer 228 that opposes the first planar surface 232 may be disposed and come into contact with.

The housing 202 may also include a fifth portion 314 configured to engage and support at least a portion of the proximal portion 224 of the coupling member 214. For some example configurations, the fifth portion 314 of the housing 202 and the proximal portion 224 of the coupling member 214 may be configured to be threadingly engaged with each other. During assembly, the second planar surface 244 of the washer 228 may be positioned on the inner surface 312, and then the proximal portion 224 of the coupling member 214 may be inserted through the hole of the metal washer 228 and screwed into the fifth portion 314 until the shoulder 230 of the coupling member 214 securely biases the washer 228 against the inner surface 312.

The housing 202 may further include a sixth portion 316 that houses or surrounds the coupling member 214 and a proximal portion of the needle body 132, including the proximal needle hub 218. The sixth portion 316 may distally extend to the portion including the first and second members 148, 150 that move the elongate tubular member 124 relative to the needle (Fig. 1A). In addition, as shown in Fig. 4, the housing 202 may include inner surfaces 318, 320 that define portions of the fluid delivery channel 204, particularly those portions not defined by the luer and coupling member lumens 220, 222.

For some example configurations, distal ends of the conductive pins 234a-234c may be mounted or secured in a base 322. The housing 202 may further include a seventh portion 324 that includes holes 326 coaxially aligned with areas 328 of the base 322 where the conductive pins 234a-234c are mounted. From the base 322, the pins 234a-234c may proximally extend in the holes 326. Proximal ends of the pins 234a-234c may proximally extend beyond the holes to outside of the housing 202. The side view of Figs. 3 and 4 shows only two of the three pins, 234a, 234b extending in the holes 326. As described in further detail below, a surface 325 of the seventh portion 324 and the proximal ends of the pins 234a-234c may be integrated together to form a handle-side connection area 327 adapted for removable connection with the electrical cable assembly 122.

An internal channel or passageway 330 may extend in the housing 202 from the coupling member 214 and the washer 228 to the base 322. As shown in Fig. 4, the wire 236 electrically connecting the washer 228 to the first pin 234a may extend in the passageway 330.

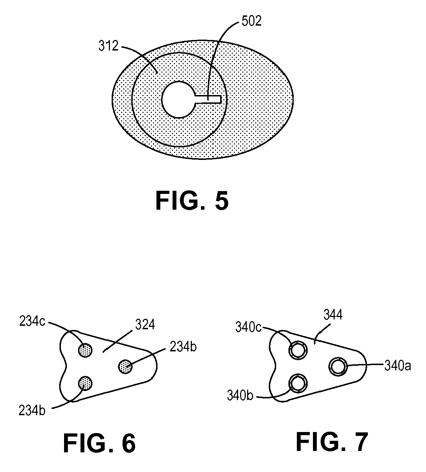

Referring to Fig. 5, a cross-sectional axial view taken along lines 5-5 in Fig. 3 is shown. As shown in Fig. 5, a gap 502 may extend in the inner surface 312 so that the portion of the washer 228 that is connected to the wire 236 (e.g., the second planar surface 232) is in communication with the internal channel 330. As such, the wire 236 may extend from the washer 228 through the gap 502 and then in the internal channel 330 toward the base 322.

Referring back to Fig. 4, the proximal portion of the thermocouple 142 may also extend in the internal channel 330. In particular, the proximal portion of the thermocouple 142 may extend through the gap 238 of the coupling member 214 and then through the internal channel 330 to the base 322. Proximal ends of the wire 236 and conductors of the thermocouple 142 may be connected to respective contacts 332 of the base 322. The base 322 may further include conductive members 334 that electrically connect the contacts 332 and the conductive pins 234a-234c. Ways other than use of the base 322 and its electrical contacts 332 to electrically connect the wire 236 and proximal conductor ends of the thermocouple 142 may be possible.

Also, as shown in Fig. 4, a sealant 336 may be disposed in the internal channel 330 to prevent fluid that enters the internal channel 330 from the fluid delivery channel 204 from reaching the base 322 where the electrical connections are made and damaging those connections. An example sealant may be epoxy, although other types of sealants may be possible.

In addition, the electrical cable assembly 122 may be removably connectable with the handle assembly 120. In particular, the electrical cable assembly 122 may include a connector 338 that has cable-side contacts comprising conductive sockets or receptacles 340a, 340b, 340c configured to removably engage and form an electrical connection with the conductive pins 234a-234c. (The second receptacle 340b is not shown in Figs. 3 and 4). The connector 338 may include a housing 344. A portion 345 of the housing 344 may be integrated with the receptacles 340a-340c to form a cable-side connection area 347 that is adapted for removable connection with the handle-side connection area 327. In this way, the conductive pins 234a-234c projecting from the housing 202 and the conductive receptacles 340a-340c of the electrical cable assembly 122, and in general the handle-side and cable-side connection areas 327, 347, may form a plug and socket configuration. Other ways to configure the handle-side and cable side connection areas 327, 347 in order for the areas 327, 347 to be adapted to mate with each other and form removably physical and electrical connections may be possible. For example, the plug and socket configuration shown in Figs. 2-4 may be reversed. That is, the handle assembly 120 may include receptacles and the electrical cable assembly 122 may include pins. Handle-side and cable-side electrical contacts other than pins and receptacles and that are adapted for removable connectable with each other may alternatively be used. One example type of electrical contact may include magnets. Regardless of how the handle-side and cable-side contacts are configured to form the removable connection, by integrating the cable-side contacts 340a, 340b, 340c with a single connector 338 and its housing 344, only a single connection between the handle assembly 120 and the electrical cable assembly 122 may be made in order for the handle assembly to be configured to electrical communication with both the power source 108 and the temperature measurement device 110 via the cable assembly 122.

In addition, the electrical cable assembly 122 may include a first elongate conductive member 342, which may include a single wire or conductive path, that is connected to and extends from the first conductive receptacle 340a within a housing 344 of the connector 338. The first conductive member 342 may further extend to outside the connector housing 344 and terminate with a first plug 346 or other connector adapted to be removably connected to the power source 108.

The electrical cable assembly 122 may also include a second elongate conductive member 348, which may include two wires or conductive paths. The second conductive member 348 may be connected to and extend from the second and third conductive receptacles 340b, 340c within the connector housing 344. Each of the conductive paths may be connected to a different one of the receptacles 340b, 340c. In addition, each of the conductive paths of the first and second elongate conductive members 342, 348 may be electrically insulated from each other. Also, the second conductive member 348 may further extend to outside the connector housing 344 and terminate with a second plug 350 or other connector adapted to be removably connected to the temperature measurement device 110. As shown in Figs. 3 and 4, the second plug 350 may include a first lead 352 connected with a first conductive path of the second conductive member 348 and a second lead 354 connected with a second conductive path of the second conductive member 348.

The electrical cable assembly 122 may additionally include an outer sheath 356 that encases the first and second elongate conductive members 342, 348 together outside of the connector housing 344. As shown in Figs. 3 and 4, the outer sheath 356 may not extend the entire lengths of the first and second elongate conductive members 342, 348 so that each of the first and second elongate conductive members 342, 348 have sufficient length to extend independently of each other such that the first and second plugs 346, 350 may be connected to the power source 108 and the temperature measurement device 110, respectively.

Fig. 6 shows a cross-sectional axial view of the seventh portion 324 of the handle-side connection area 327, including the handle assembly housing portion 325 and proximal ends of the pins 234a-234c, taken along lines 6-6 of Fig. 4. Fig. 7 shows a cross-sectional axial view of the cable-side connection area 347, including the connector housing portion 345 and the receptacles 340a-340c, taken along lines 7-7 of Fig. 4. Together, Figs. 6 and 7 show an example arrangement or orientation of the pins 234a-234c and conductive receptacles 340a-340c to form the plug and socket configuration.

As shown in Fig. 7, as well as in Figs. 3 and 4, the first conductive receptacle 340a that electrically connects the power source 108 with the handle assembly 120 and the second and third conductive receptacles 340b, 340c that electrically connect the temperature measurement device 110 with the handle assembly 120 may be integrated together in the same connector housing 344. As such, when one of the conductive receptacles 340a-340c engages with a corresponding one of the conductive pins 234a-234c, the other of the conductive receptacles 340a-340c are also engaged with their corresponding conductive pins 234a-234c.

Additionally, as shown in Figs. 6 and 7, the plug and socket configuration may have a configuration that causes the conductive receptacles 340a-340c to engage with the conductive pins 234a-234c in a single way in order to ensure that the receptacles 340a-340c are not improperly engaged or connected to pins 234a-234c with which they are not supposed to be connected to. For example, as shown in Figs. 6 and 7, the pins 234a-234c may be oriented to form a triangle, and the conductive receptacles 340a-340c may be similarly oriented to form a similarly shaped triangle. A spacing of the pins 234a-234c apart from each other, and a similar spacing of the conductive receptacles 340a-340c apart from each other may be such that in order for the three conductive receptacles 340a-340c to be engaged with the three conductive pins 234a-234c at the same time, the first conductive receptacle 340a must be engaged with the first pin 234a, the second conductive receptacle 340b must be engaged with the second pin 234b, and the third conductive receptacle 340c must be engaged with the third pin 234c. In addition or alternatively, cross-sectional shapes of handle assembly housing portion 325 and the connector housing portion 345, may be similarly shaped to align or match up with each other. Further, their alignable cross-sectional shapes may only align with each other when the receptacles 340a-340c are correctly engaged with their corresponding pins 234a-234c. For some examples, as indicated by the three-corner-rounded triangles shown in Figs. 6 and 7, the cross-sectional shapes may be symmetrical with respect to only a single axis. Other ways to ensure that the receptacles 340a-340c are properly engaged and connected to the pins 234a-234c may be possible.

In the example configuration shown in Figs. 2 and 4, the coupling member 214 may be made of a conductive material and form part of the RF energy delivery path. For these configurations, RF energy may be delivered to the needle 130 via the coupling member 214. In other words, when the needle body 132 is coupled to the coupling member 214, the needle 130 is both in fluid communication with the fluid delivery path 204 and in electrical communication with the RF delivery path of the handle assembly 120.

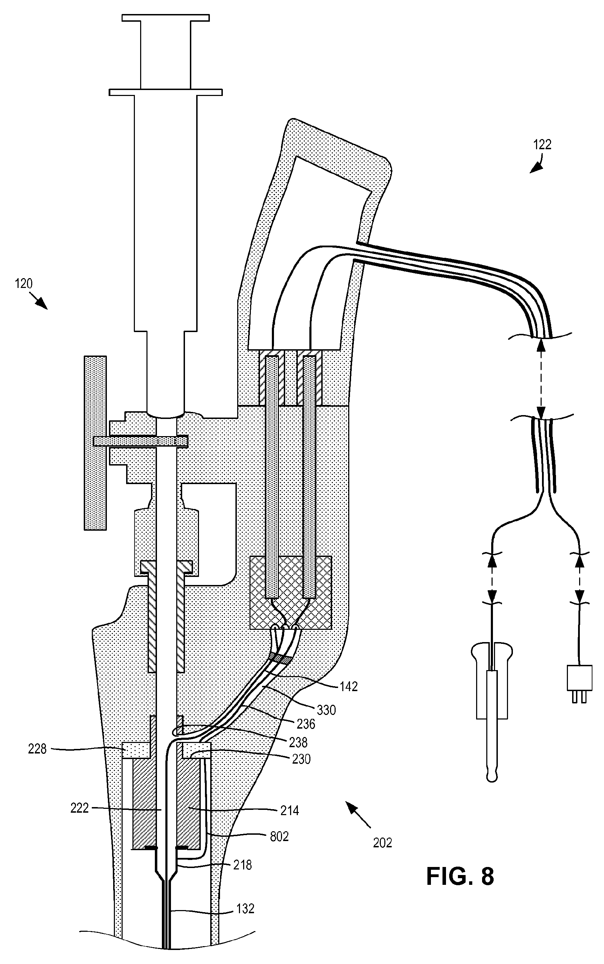

Various other ways of configuring the RF energy delivery path and/or electrically coupling the needle body 132 to the RF delivery path of the handle assembly 120 may be possible. For example, Fig. 8 is a cross-sectional side view of another example configuration of the handle assembly 120. The handle assembly components shown in Fig. 8 is similar to those shown in Fig. 4, except that the coupling member 214 may be made of a non-conductive material and/or not be part of the RF energy delivery path of the handle assembly 120. For this other example configuration, a second wire or other conductive element 802 may connect the washer 228 to the needle body 132 in order to electrically couple the needle body 132 to the RF energy delivery path of the handle assembly 120.

In other example configurations, the coupling member 214 may be made of a conductive material and part of the RF energy delivery path, but the washer 228 may not be made of a conductive material and/or not part of the RF energy delivery path. For these other example configurations, the wire 236 extending in the channel 330 may directly connect to the conductive coupling member 214. In still other example configurations, both the coupling member 214 and the washer 228 may not be part of the RF energy delivery path. For these configurations, the wire 236 may directly connect to the needle body 132. For example, the wire 236 may extend through the gap 238 and the coupling member lumen 222 of the coupling member 214 along with the thermocouple 142, and then connect to the needle body 132 distal the coupling member 214. In other configurations, the washer 228 may be sized so that the wire 236 can extend around or outside of the washer 228 and the coupling member 214 so that the wire 236 can directly connect to the needle body 132, similar to the path taken by the second wire 802 shown in Fig. 8. Various other ways of electrically coupling the needle body 132 to the RF energy delivery path of the handle assembly 120 may be possible.

In addition, ways to couple the needle body 132 to the housing 202 other than through the coupling member 214 and/or the washer 228 may be possible. For example, in other configurations, the coupling member 214 and the washer 228 may be a single, integral component. In other example configurations, the washer 228 may not be included as a component of the handle assembly 120. In other example configurations, the needle body 132 may directly couple to the housing 202 without the use of the coupling member 214 and/or the washer 228. Other configurations may be possible.

A method of ablating a cyst within a patient using the electrosurgical system 100 described with reference to Figs. 1A and 2-7 is now described. Referring to Fig. 1A, the distal portion 118 of the elongate insertion portion 114 of the electrosurgical device 106 may be delivered to a location 156 of the patient's GI tract near or determined to be desirable for accessing the cyst 102. Example GI tract locations 156 may be the stomach or the duodenum, although other GI tract locations near or adjacent to where a cyst may be accessed from the GI tract may be possible. In addition, the distal portion 118 may be delivered endoscopically. That is, a distal portion of an endoscope (not shown) may first be delivered to the GI tract location 156, such as by being inserted into the patient's mouth and distally advanced through the esophagus and then further to the GI tract location 156. The distal portion 118 of the insertion portion 114 may then be delivered through a working channel of the endoscope until it reaches the GI tract location 156. As shown in Fig. 1A, when the distal portion 118 is delivered to and initially arrives at the GI tract location 156, the needle 130, including the distal tip 136, may be disposed or retracted within the central lumen 128 of the elongate tubular member 124.

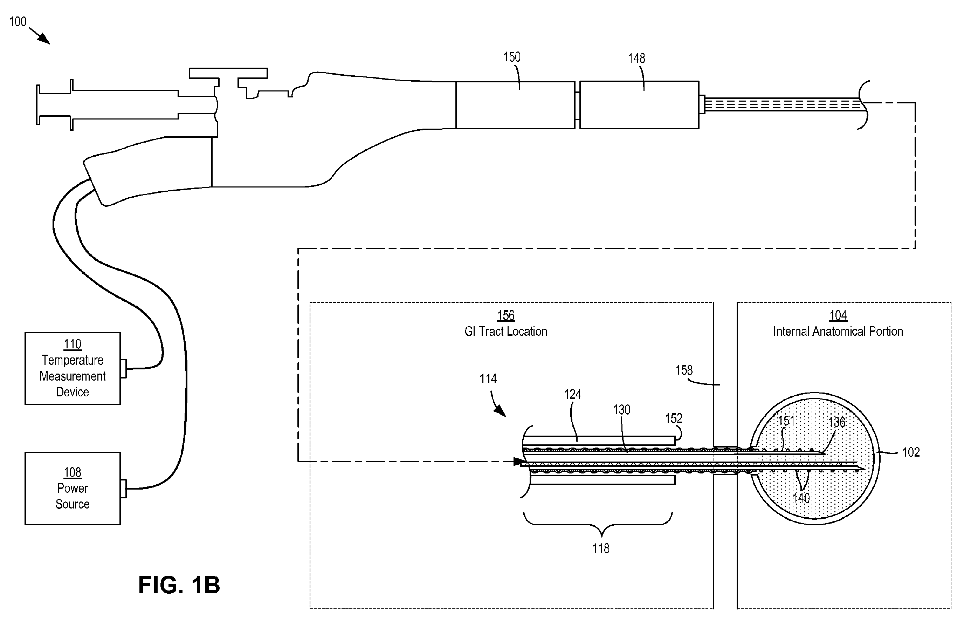

Referring to Fig. 1 B, once at the GI tract location 156, the first and second members 148, 150 of the handle assembly 120 may be operated to distally advance the distal portion 151, including the distal tip 136, past the distal end 152 of the elongate tubular member 124 in order to expose the distal portion 151 to outside the elongate tubular member 124. The distal portion 118 of the insertion portion 114 may then be distally advanced such that the distal tip 136 punctures a wall 158 of the GI tract and proceeds further into the anatomical portion 104 and into the cyst 102. During this time, an ultrasound transducer coupled to a distal end of the endoscope (not shown) may be activated in order to generate ultrasound images, which in turn may provide ultrasound guidance and/or visualization as the distal portion 151 of the needle 130 is advanced into the cyst 102. The dimples 140 may enhance the ultrasound guidance and/or visualization in order to facilitate the process.

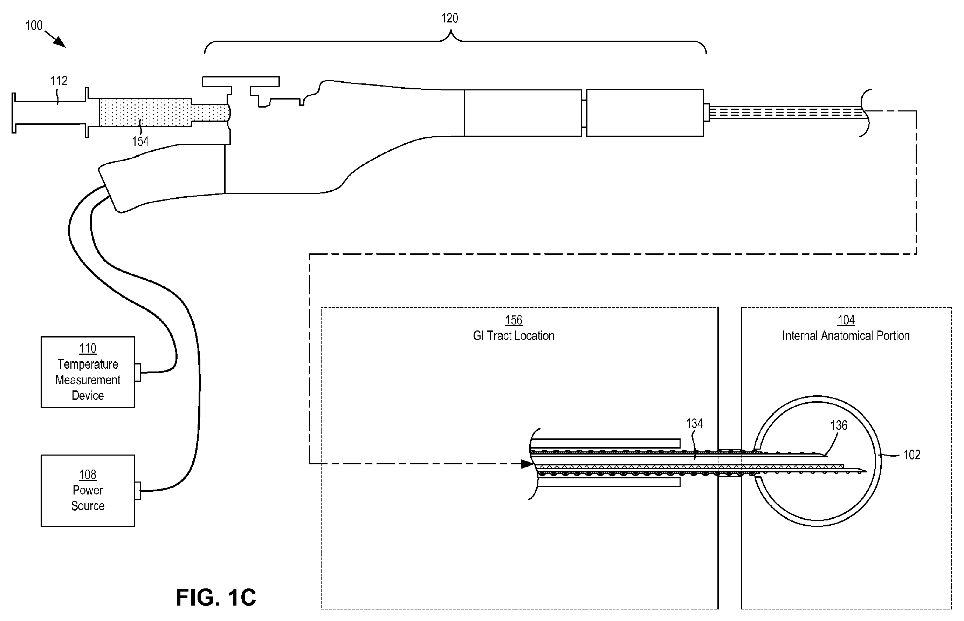

Referring to Fig. 1C, the syringe 112 may be operated in order to cause the cyst fluid 154 to be aspirated through distal tip 136 and the needle lumen 134 and into the syringe 112 via the handle assembly 120. Referring to Fig. 4, while flowing through the handle assembly 120, the cyst fluid 154 may pass through the fluid delivery channel 204, as defined by the coupling member lumen 222 and the luer lumen 220, as well as portions 318, 320 of the housing 202.

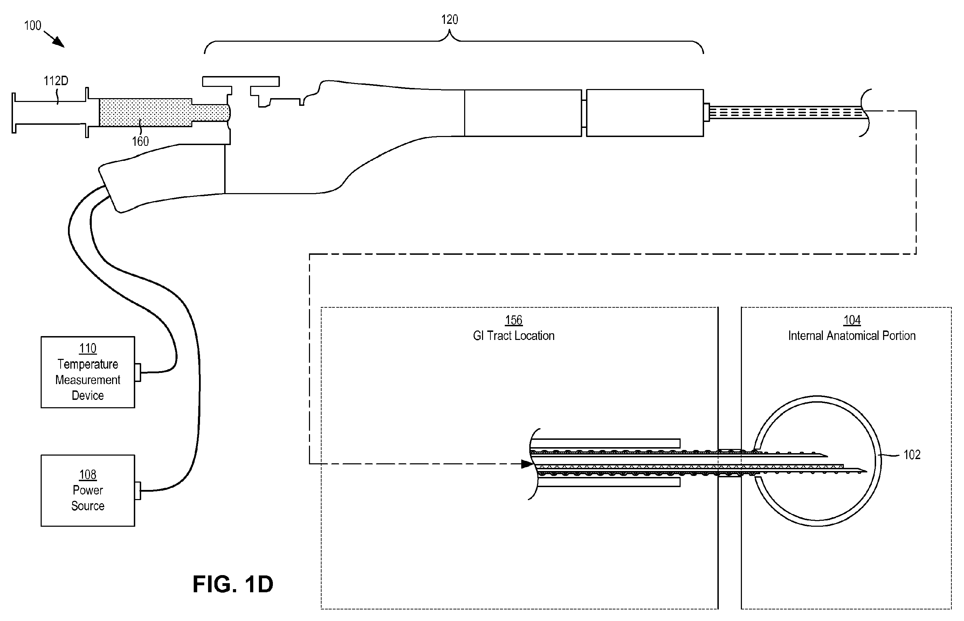

Referring to Fig. 1D, after the cyst fluid 154 is drained from the cyst 102, the initial syringe 112 filled with the cyst fluid 154 may be detached from the handle assembly 102 and a syringe 112D filled with a conductive fluid 160, such as saline, may be attached to the handle assembly 120. The syringe 112D may the same as or different from the initial syringe 112 that aspirated the cyst fluid. In addition, in some example methods, the syringe 112D filled with the conductive fluid may be attached to the handle assembly 120 after a determination to ablate the cyst 102. The determination may be made based on an analysis of the cyst fluid 154 that was aspirated into the initial syringe 112.

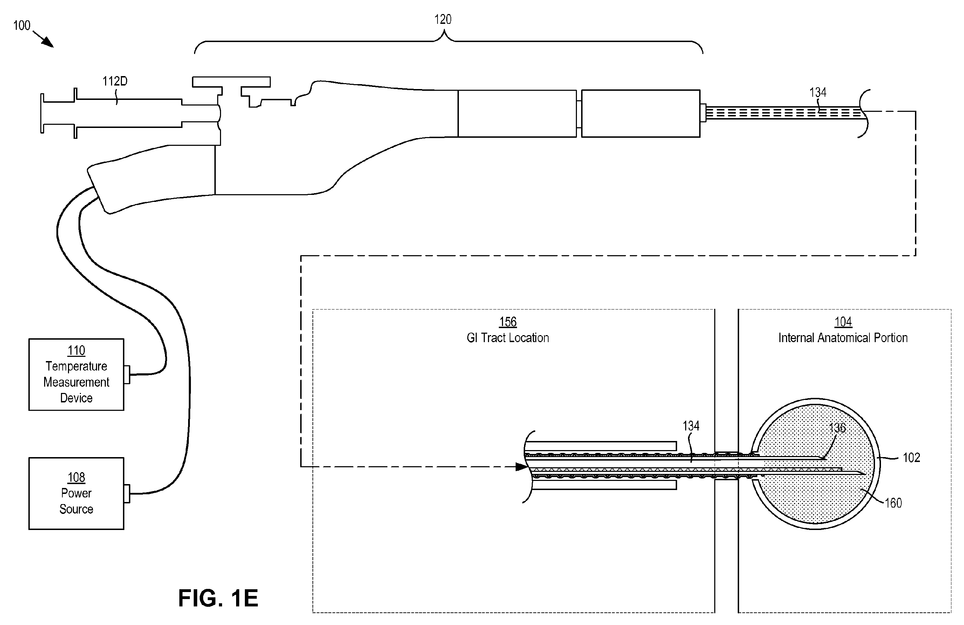

Referring to Fig. 1 E, the syringe 112D may be operated to expel the conductive fluid 160 into the handle assembly 120. The conductive fluid 160 may pass through the fluid delivery path 204 of the handle assembly 120 (Figs. 2-4) and into the needle lumen 134. The conductive fluid 160 may continue to flow through the needle lumen 134 and then past the distal tip 136 to within the cyst 102 in order to fill the cyst 102 with the conductive fluid 160.

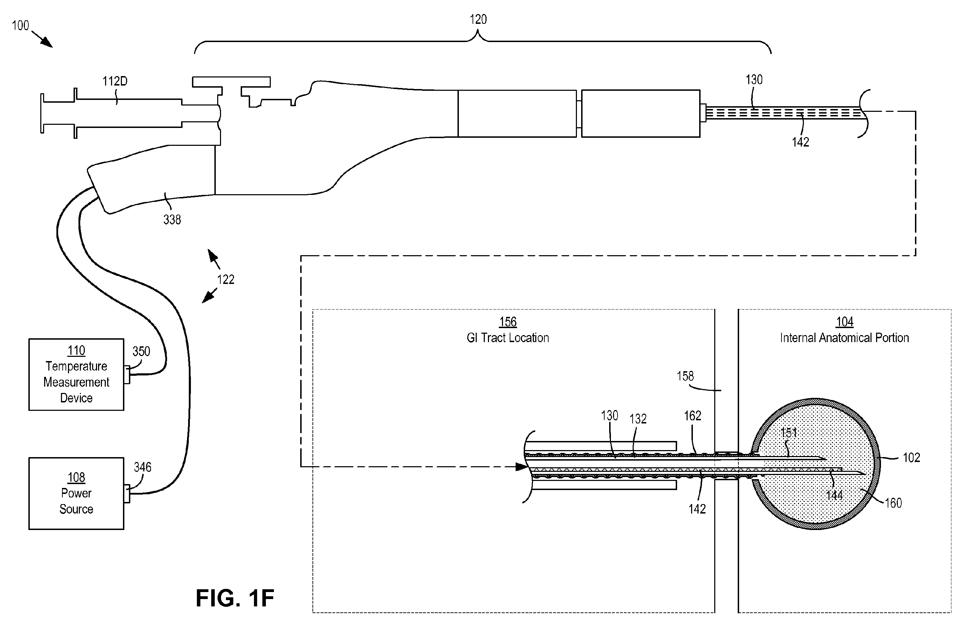

Referring to Fig. 1 F, after the conductive fluid 160 has been expelled from the syringe 112D and into the cyst 102, the cyst 102 may be ablated through activation of the power source 108. As shown in Fig. 1 F, the first plug 346 may be connected to the power source 108 and the second plug 350 may be connected to the temperature measurement device 110. Further, as shown in Fig. 1 F, the connector 338 of the electrical cable assembly 122 may be connected with the handle assembly 120. In this configuration, the distal portion 151 of the needle 130 may be in electrical communication with the power source 108 and the thermocouple 142 may be in electrical communication with the temperature measurement device 110 via the handle assembly 120 and the electrical cable assembly 122.

When the power source 108 is activated, RF energy may be delivered from the power source 108, through the electrical cable assembly 122, through RF energy delivery path of the handle assembly 120, and to the needle body 132. For the handle assembly configurations shown in Figs. 2-8, the RF energy may flow through the first plug 346, through the first elongate conductive member 342, through the first conductive receptacle 340a and the first conductive pin 234a engaged with the first conductive receptacle 340a, through the wire 236 to the needle body. For particular configurations where the conductive washer 228 and the coupling member 214 are part of the RF energy delivery path, the RF energy may flow through the conductive washer 228 and the coupling member 214 to reach the proximal hub 218 of the needle body 132 (Figs. 2-4). The RF energy may flow through needle body 132 to the distal portion 151 located inside the cyst 102 and surrounded by the conductive fluid 160. Since the fluid 160 is conductive, the RF energy may flow from the distal portion 151 to the cyst 102 via the conductive fluid 160. The RF energy may heat up the cyst 102, eventually ablating the cyst 102, as denoted by the darkening of the cyst 102 in Fig. 1 F. In this example method, the RF energy may be applied in a monopolar fashion. Although not shown, a backplate may be attached to the patient and also connected to a return wire that may return back and be connected to the power source 108.

So that only the cyst 102 and not other portions of the patient (e.g., the GI tract location 156, the GI wall 158, or the internal anatomical portion 104) receive the RF energy, the portion of the needle 130 that is inserted into the cyst 102 may be exposed or uninsulated, and at least some of the needle 130 proximal the portion that is exposed or uninsulated may be covered or coated with an insulating material 162, such as parylene as an example. How much of the distal portion 151 is uninsulated may depend on the size of the cyst 102. In general, any portion of the needle 130 that is not inserted into the cyst 102 but that may still be exposed to outside of the elongate tubular member 124 may be covered with the insulating material 162. In some example configurations, a length of the exposed or uninsulated portion may be about one centimeter.

While RF energy is being applied to the cyst 102, the distal end 144 of the thermocouple 142 may be sensing the temperature within the cyst 102 and surrounding the distal end 144. In response to the sensed temperature, the two conductors comprising the distal end 144 may generate a voltage between them, which may be proximally transmitted through the thermocouple 142 as a temperature signal. The temperature signal may be communicated through the thermocouple 142, through the second and third conductive pins 234b, 234c, through the second and third receptacles 340b, 340c engaged with the second and third conductive pins 234b, 234c, communicated through the second elongate conductive member 348, through the second plug 350, and to the temperature measurement device 110. Based on the received temperature signal, the temperature measurement device 110 may be measure a temperature sensed by the distal end 144 of thermocouple 142. In some methods, the temperature measurement device 110 may be monitored to determine when the sensed temperature in or around the cyst 102 reaches a predetermined temperature level. The sensed temperature reaching the predetermined temperature level may indicate that the cyst 102 has been successfully ablated and application of RF energy in the internal anatomical portion 104 should stop. For some example methods, the predetermined temperature level may be fifty-five degrees Celsius.

In addition or alternatively, activation of the power source 108 and/or application of the RF energy to the cyst 102 may cease after a predetermined threshold amount of RF energy has been applied to the cyst 102. The predetermined amount of RF energy may correspond to an amount that is sufficiently large enough to ablate the cyst 102 and small enough so as not to excessively heat the internal anatomical portion 104 and/or cause harm to the patient. For ablation of cysts, an example predetermined amount of RF energy may be in a range from 200 Joules to 10,000 Joules.

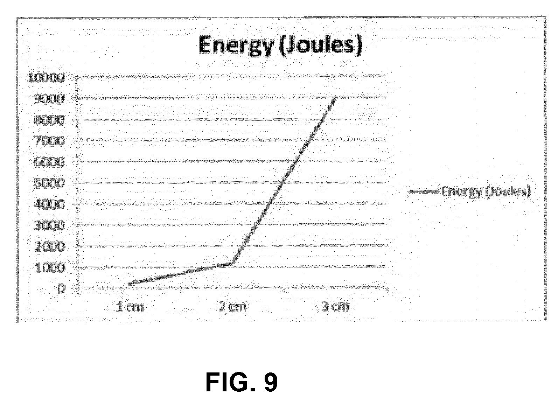

Additionally, the predetermined amount of RF energy may vary from cyst to cyst and may depend on the size or diameter of the cyst. Fig. 9 shows a plot of the predetermined amount of energy as a function of the diameter of the cyst. Points making up the plot were generated by selecting known power settings in Watts for the power source 110 and measuring the amount of time it took for the temperature within the cyst 102, as sensed by the distal end 144 of the thermocouple 142, to reach 55 degrees Celsius. An example exponential best-fit curve having a mathematical function that corresponds to the plot shown in Fig. 9 may be represented by the following equation:

$$y = 32.618e^{1.8557x}$$

where y is the predetermined amount of RF energy and x is the size of the cyst in centimeters.

As indicated in the plot shown in Fig. 9, for cysts having a diameter of about one centimeter, the predetermined amount of RF energy may be about 220 Joules; for cysts having a diameter of about two centimeters, the predetermined amount of RF energy may be about 1,200 Joules; and for cysts having a diameter of three centimeters, the predetermined amount of RF energy may be about 9,000 Joules. Using a 25% tolerance, ranges for predetermined amounts of RF energy to be applied to cysts may be identified for different cyst sizes. As examples, when the cyst has a diameter of about one centimeter, the predetermined amount of RF energy applied to the cyst may be between 165 Joules and 275 Joules; when the cyst has a diameter of about two centimeters, the predetermined amount of RF energy applied to the cyst may be between 900 Joules and 1500 Joules; and when the cyst has a diameter of about three centimeters, the predetermined amount of RF energy applied to the cyst may be between 6,750 Joules and 11,250 Joules.

As previously described, the size or diameter of the cyst may be determined from initial CT scans performed prior to the ablation. Based on the determined size, selecting a known power setting (in Watts), and then using the plot in Fig. 9 or the above equation, the amount of time to ablate a given cyst having a diameter in a range of about one centimeter to three centimeters may be determined. Then when RF energy is applied to the cyst, the amount of time the RF energy is being applied may be monitored, either by a time monitoring device or a person involved in performance of the procedure, and application of the RF energy to the cyst may be stopped once the time period has expired. In addition or alternatively, a device configured to calculate the amount of RF energy delivered to the cyst may determine when to cease application of the RF energy by the determining when the amount of the RF energy delivered to the cyst has reached the predetermined amount. In either case, use of the thermocouple 138 and the temperature measurement device 110 may not be needed if the plot in Fig. 9 and/or the above equation are implemented to determine when application of the RF energy to the cyst should be stopped.

The above-described ablation procedure may be performed to remove a cyst that a physician determines to remove (e.g., because it is too large or malignant) and as an alternatively to invasive or open surgery that involves cutting into the patient's abdominal cavity from the patient's skin. In addition or alternatively, the above-described ablation procedure may be performed prophylactically in the sense that it may be performed to remove the cyst before the cyst has grown to a size necessitating removal.

In addition, the above-described ablation procedure may be performed with an electrosurgical medical system other than the system 100 and/or the particular configurations of the handle and power cord assemblies 120, 122 shown in Figs. 1A-8. For example, handle and power cord assemblies that do not utilize a removable connection and/or a connector assembly that uses separate, multiple connections to electrically couple the power source 108 and the temperature measurement device 110 to the electrosurgical medical device may be used to perform the above-described ablation procedure.

In addition, the configurations of the handle assembly 120 and the electrical cable assembly 122 shown and described with reference to Figs. 2-8 are not limited for use with only electrosurgical devices that ablate cysts. Rather, the handle and electrical cable assemblies 120, 122 may be used more generally with any electrosurgical devices that use an elongate tubular conductive member to deliver both fluid and electrosurgical or RF energy to a treatment site, and that sense temperature at the treatment site.

Additionally, in many cases, electrosurgical devices may generally be one-time or limited use devices that are discarded after an electrosurgical procedure is performed. By configuring the electrical cable assembly 122 to be removably connectable with the handle assembly 120, the electrical cable assembly 122 may be detached or disconnected from the handle assembly 120 after the procedure is performed and before the rest of the electrosurgical device is discarded. The electrical cable assembly 122 may then be used with a different electrosurgical device and/or attached to a different handle assembly 120 for another procedure. As such, the removably connectable electrical cable assembly 122 provides a single, reusable cable assembly that can be simultaneously connected to both a power source that generates the RF energy and a temperature measurement device, and that can also be used with multiple electrosurgical devices for multiple procedures. Because the cable assembly 122 is reusable and not fixedly attached to the handle assembly 122, the electrical cable assembly 122 does not have to be discarded after a procedure is performed. As such, less of a single electrosurgical device is discarded after a procedure is performed.

Additionally, the removably connectable features of the handle assembly 120 and the cable assembly 122 may be used for electrosurgical medical devices that are connected to the power source 108 and the temperature measurement device 110 in order to deliver RF energy and sense temperature, respectively, but that do not communicate fluid, at least not through the elongate conductive member that receives RF energy. Handle assemblies for these other configurations may include the removably connectable features of the handle assembly 120 shown in Figs. 2-8, but may not include a fluid delivery path 204. In addition, for these other configurations, the elongate conductive member that receives RF energy may not necessarily be a hollow structure capable of communicating fluid.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

The following numbered clauses set out further important features of the disclosure:

1. An electrosurgical medical device comprising an elongate conductive member extending from a proximal portion to a distal portion; a thermocouple; a handle assembly coupled to the elongate conductive member, the handle assembly comprising: a handle assembly housing; a radio frequency (RF) energy delivery path extending within the handle assembly housing and electrically coupled to the elongate conductive member; and a plurality of handle-side electrical contacts; and an electrical cable assembly comprising:

a connector housing; and a cable-side connection area that comprises a plurality of cable-side electrical contacts integrated with the connector housing, wherein the plurality of cable-side electrical contacts are configured for removable connection with the plurality of handle-side contacts, and when the plurality of cable-side contacts are connected to the plurality of handle-side contacts, the electrical cable assembly is electrically coupled to the RF energy delivery path and the thermocouple.

2. The electrosurgical medical device of clause 1, the handle assembly further comprises: a handle-side connection area adapted for removable connection with the cable-side connection area, the handle-side connection area comprising a portion of the handle assembly housing and the plurality of handle-side electrical contacts integrated with the portion of the handle assembly housing.

3. The electrosurgical medical device of clause 2, wherein the handle-side connection area and the cable-side connection area form a plug and socket configuration.

4. The electrosurgical medical device of clauses 2 or 3, wherein the handle-side connection area and the cable-side connection area comprise matching cross-sectional shapes that are symmetrical with respect to a single axis.

5. The electrosurgical medical device of any of clauses 2 through 4, wherein the electrical cable assembly further comprises: a first elongate conductive member configured to be electrically coupled to the RF energy delivery path and terminating with a first connector adapted for removable connection with a power source configured to generate the RF energy; and a second elongate conductive configured to be electrically coupled to the thermocouple and terminating with a second connector adapted for removable connection with a temperature measurement device.

6. The electrosurgical medical device of any of clauses 1 to 5, wherein the handle assembly further comprises a fluid delivery channel extending within the handle assembly housing, and wherein the elongate conductive member is electrically coupled to the RF energy delivery path and in fluid communication with the fluid delivery channel.

7. The electrosurgical medical device of clause 6, wherein the handle assembly further comprises a coupling member that couples the elongate conductive member to the handle assembly housing, wherein the coupling member comprises a coupling member lumen that forms part of the fluid delivery channel.

8. The electrosurgical medical device of clause 7, wherein the coupling member comprises a conductive material and forms part of RF energy delivery path, the elongate conductive member being both electrically coupled to the RF energy delivery path and in fluid communication with the fluid delivery channel via the coupling member.

9. The electrosurgical medical device of clauses 7 or 8, wherein the thermocouple extends through at least a portion of the coupling member lumen.

10. The electrosurgical medical device of any of clauses 1 through 9, wherein the elongate tubular member comprises a needle.

## Claims

1. An electrosurgical medical device comprising:

    a handle assembly comprising:

        a handle assembly housing;
        a fluid delivery channel extending within the handle assembly housing; and
        a radio frequency (RF) energy delivery path extending within the handle assembly housing;

        and
        an elongate tubular member extending from a proximal portion to a distal portion, the elongate tubular member comprising a conductive body and a lumen extending in the conductive body, wherein the conductive body is electrically coupled to the RF energy delivery path and the lumen is in fluid communication with the fluid delivery channel.

2. The electrosurgical medical device of claim 1, further comprising a coupling member that couples the conductive body to the handle assembly housing, wherein the coupling member comprises a coupling member lumen that forms part of the fluid delivery channel.

3. The electrosurgical medical device of claim 2, wherein the coupling member comprises a conductive material and forms part of RF energy delivery path, the elongate tubular member being both electrically coupled to the RF energy delivery path and in fluid communication with the fluid delivery channel via the coupling member.

4. The electrosurgical medical device of claims 2 or 3, wherein the handle assembly further comprises a conductive donut-shaped member that is part of the RF energy delivery path and disposed about a proximal portion and in contact with a shoulder of the coupling member.

5. The electrosurgical medical device of any of claims 2 through 4, further comprising a thermocouple comprising a distal end disposed at a distal portion of the elongate tubular member and proximally extending to within the handle assembly housing, wherein the thermocouple extends through at least a portion of the coupling member lumen.

6. The electrosurgical medical device of claim 1, further comprising a thermocouple comprising a distal end disposed at a distal portion of the elongate tubular member and proximally extending to within the handle assembly housing.

7. The electrosurgical medical device of claims 5 or 6, wherein the handle assembly further comprises a handle-side connection area adapted for removable connection with an electrical cable assembly, the handle-side connection area comprising a portion of the handle assembly housing and a plurality of handle-side electrical contacts integrated with the portion of the handle assembly housing, wherein the plurality of handle-side electrical contacts comprises a first handle-side contact that is part of the RF energy delivery path and second and third handle-side contacts that are in electrical communication with the thermocouple.

8. The electrosurgical medical device of claim 7, further comprising a second channel that extends in the handle assembly housing, wherein the thermocouple and a conductive wire that is part of the RF energy delivery path extend in the second channel.

9. The electrosurgical medical device of claim 8, wherein the handle assembly comprises a base that supports the plurality of handle-side electrical contacts, and wherein the second channel extends in the housing to the base.

10. The electrosurgical medical device of claims 8 or 9, wherein the handle assembly further comprises a conductive donut-shaped member that is part of the RF energy delivery path, wherein the conductive donut-shaped member comprises a planar surface biased against an internal surface of the handle assembly housing, and wherein the conductive wire extends through a gap in the internal surface in order to be connected to the conductive donut-shaped member.

11. The electrosurgical medical device of any of claims 7 through 10, further comprising the electrical cable assembly, wherein the electrical cable assembly comprises:

   a connector comprising a connector housing;
   a cable-side connection area adapted for removable connection with the handle-side connection area, wherein the cable-side connection area comprises a portion of the connector housing integrated with a plurality of cable-side electrical contacts,

   wherein the plurality of cable-side electrical contacts comprises a first cable-side contact designated for removable connection with the first handle-side contact, and second and third cable-side contacts designated for removable connection with the second and third handle-side contacts.

12. The electrosurgical medical device of claim 11, wherein the handle-side connection area and the cable-side connection area form a plug and socket configuration.

13. The electrosurgical medical device of claims 11 or 12, wherein the handle-side connection area and the cable-side connection area comprise matching cross-sectional shapes that are symmetrical with respect to a single axis.

14. The electrosurgical medical device of any of claims 11 through 13, wherein the electrical cable assembly further comprises:

   a first elongate conductive member electrically connected to the first cable-side contact, the first elongate conductive member terminating with a first plug adapted for removable connection with a power source configured to generate the RF energy; and
   a second elongate conductive member electrically connected to the second and third cable-side contacts, the second elongate conductive member terminating with a second plug adapted for removable connection with a temperature measurement device.

15. The electrosurgical medical device of any of claims 1 through 14, wherein the elongate tubular member comprises a needle.

**FIG. 1A**

100

148

150

Internal Anatomical Portion
104

102

136

151

140

158

GI Tract Location
156

152

130

124

118

114

110
Temperature
Measurement
Device

108
Power
Source

FIG. 1B

16

FIG. 1C

FIG. 1D

EP 3 087 943 A1

FIG. 1E

**FIG. 1F**

EP 3 087 943 A1

FIG. 2

**FIG. 3**

**FIG. 4**

**FIG. 5**

**FIG. 6**

**FIG. 7**

122

120

228

222

142
330
236
238
230

214
802
218
132

202

**FIG. 8**

**FIG. 9**

Europäisches
Patentamt

European
Patent Office

Office européen
des brevets

# EUROPEAN SEARCH REPORT

Application Number

EP 16 16 7580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2006/253183 A1 (THAGALINGAM ARAVINDA [AU] ET AL) 9 November 2006 (2006-11-09)<br>* paragraphs [0090] - [0096]; figures 2b, 3a * | 1-3,5,6, 15<br>7-14<br>4 | INV.<br>A61B18/14<br><br>ADD.<br>A61B18/00 |
| X<br>Y | US 5 314 406 A (ARIAS JUAN J [US] ET AL) 24 May 1994 (1994-05-24)<br>* column 1, lines 6-10; figures 1a, 1b *<br>* column 7, line 53 *<br>* column 9, lines 1-31 *<br>* column 10, lines 57-66 * | 1-4,15<br>7-14 | |
| Y | US 5 545 200 A (WEST SCOTT H [US] ET AL) 13 August 1996 (1996-08-13)<br>* column 11, lines 6-9; figure 1 * | 7-14 | |

TECHNICAL FIELDS
SEARCHED (IPC)

A61B

The present search report has been drawn up for all claims

| Place of search | Date of completion of the search | Examiner |
|---|---|---|
| Munich | 15 September 2016 | Aronsson, Fredrik |

EPO FORM 1503 03.82 (P04C01)

**ANNEX TO THE EUROPEAN SEARCH REPORT
ON EUROPEAN PATENT APPLICATION NO.**

EP 16 16 7580

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2016

| Patent document cited in search report | | | Publication date | Patent family member(s) | | | Publication date |
|---|---|---|---|---|---|---|---|
| US 2006253183 | A1 | | 09-11-2006 | EP | 1587426 | A1 | 26-10-2005 |
| | | | | US | 2006253183 | A1 | 09-11-2006 |
| | | | | WO | 2004043272 | A1 | 27-05-2004 |
| US 5314406 | A | | 24-05-1994 | AU | 5320494 | A | 09-05-1994 |
| | | | | US | 5314406 | A | 24-05-1994 |
| | | | | US | 5429596 | A | 04-07-1995 |
| | | | | US | 5549547 | A | 27-08-1996 |
| | | | | WO | 9408638 | A2 | 28-04-1994 |
| US 5545200 | A | | 13-08-1996 | AU | 670894 | B2 | 01-08-1996 |
| | | | | AU | 683603 | B2 | 13-11-1997 |
| | | | | AU | 7052296 | A | 23-01-1997 |
| | | | | AU | 8150394 | A | 30-05-1996 |
| | | | | CA | 2138236 | A1 | 23-05-1996 |
| | | | | DE | 9420821 | U1 | 27-04-1995 |
| | | | | US | 5545200 | A | 13-08-1996 |

EPO FORM P0459

For more details about this annex : see Official Journal of the European Patent Office, No. 12/82